# FASCICULE DE BREVET EUROPEEN

(11) **EP 1 469 823 B1**
(45) Date de publication et mention de la délivrance du brevet: **16.01.2008**
(21) Numéro de dépôt: 03712290.0
(22) Date de dépôt: 31.01.2003
(51) Int. Cl.: A61K 8/67, A61K 8/73, A61K 31/07, A61Q 19/08, A61Q 19/00

(54) **UTILISATION COSMETIQUE OU DERMATOLOGIQUE DE LA VITAMINE A OU DE SES ESTERS, EN ASSOCIATION AVEC UNE BETA-CYCLODEXTRINE PARTIELLEMENT METHYLEE**
KOSMETISCHE ODER DERMATOLOGISCHE VERWENDUNG VON VITAMIN A ODER DEREN ESTERN IN KOMBINATION MIT EINEM TEILWEISE METHYLIERTEN BETA-CYCLODEXTRIN
COSMETIC OR DERMATOLOGICAL USE OF VITAMIN A OR ESTERS THEREOF IN COMBINATION WITH A PARTIALLY METHYLATED BETA-CYCLODEXTRIN

(30) Priorité: 01.02.2002 FR 0201241
(43) Date de publication de la demande: 27.10.2004
(73) Titulaire: LVMH RECHERCHE, 45800 St. Jean de Braye (FR); COMMISSARIAT A L'ENERGIE ATOMIQUE, 75015 Paris (FR)
(72) Inventeur: ARCHAMBAULT, Jean-Christophe, F-45130 MEUNG SUR LOIRE (FR); OUVRARD-BARATON, Françoise, F-45000 ORLEANS (FR); DJEDAINI-PILARD, Florence, F-80000 AMIENS (FR); WEISSE, Sandrine, F-35000 RENNES (FR)
(74) Mandataire: Giraud, Françoise
(86) Numéro de dépôt international: PCT/FR2003/000294
(87) Numéro de publication internationale: WO 2003/063805

(56) Documents cités:
- EP-A- 0 396 184
- EP-A- 0 649 653
- WO-A-01/19332
- FR-A- 2 484 252
- US-A- 4 371 673
- US-A- 5 472 954
- US-A- 5 484 816
- US-A- 6 024 941
- DATABASE CAPLUS [Online] CHEMICAL ABSTRACTS SERVICE, COLUMBUS, OHIO, US; 1996, GUO: "binding of vitamin a by beta-cyclodextrin and heptakis (2,6-O-dimethyl)-beta-cyclodextrin" XP002227631 accession no. STN Database accession no. 1996:71956
- PATENT ABSTRACTS OF JAPAN vol. 2000, no. 12 (C, & JP 2000 256167 A (SHISEIDO CO), 19 septembre 2000 (2000-09-19)

## Description

La présente invention concerne l'utilisation dans le domaine de la cosmétique et de la dermatologie d'associations de β-cyclodextrines partiellement méthylées et de vitamine A ou d'esters de vitamine A.

Depuis de nombreuses années, la vitamine A et ses esters, notamment l'acétate et le palmitate, sont très largement utilisés en cosmétique et en dermatologie pour améliorer l'état général de la peau. Les actions de la vitamine A sont bien connues dans ces domaines d'application. Elle régule notamment le métabolisme cellulaire de la peau et, de ce fait, préserve ou restaure un bon état physiologique cutané. La vitamine A permet ainsi d'améliorer le tonus, la fermeté et l'élasticité de la peau qui tendent à diminuer avec l'âge ou par des effets de stress. En particulier, la vitamine A ou ses esters sont couramment utilisés pour retarder l'apparition des rides ou pour en atténuer leur profondeur. En dermatologie, la vitamine A agit efficacement pour favoriser la cicatrisation de la peau ainsi que dans le traitement des peaux acnéiques.

Cependant l'utilisation de tels composés est limitée en pratique par des problèmes de stabilité, de solubilité en milieu aqueux mais aussi de pénétration dans la peau. En effet, l'utilisation de la vitamine A ou de ses esters n'a d'intérêt en cosmétique et en dermatologie que si ces actifs sont biodisponibles au niveau du derme et/ou de l'épiderme.

Les problèmes techniques rencontrés pour réaliser leur solubilisation, leur stabilisation et leur pénétration dans la peau ont donc fait l'objet de nombreux développements. Des méthodes ont été décrites pour solubiliser en milieux aqueux et stabiliser la vitamine A ou ses esters. Mais les résultats obtenus ne sont pas satisfaisants en terme de pénétration dans les couches plus profondes de l'épiderme réduisant d'autant la biodisponibilité des actifs incorporés dans les compositions cosmétiques et dermatologiques antérieures.

Les cyclodextrines α, β et γ, ci-après désignées par α-CD, β-CD et γ-CD, sont décrites depuis longtemps en tant que vecteur d'actifs de toutes sortes, capables de piéger certaines petites molécules de nature hydrophobe. En particulier, les cyclodextrines sont décrites pour capter et protéger les vitamines lipophiles et les arômes.

Il est connu de l'art antérieur, dans le document WO 9421225, des compositions cosmétiques utilisant comme agent de pénétration une β-cyclodextrine afin de former des complexes d'inclusion d'esters de vitamine A, en particulier de palmitate.

Cependant, l'utilisation de la β-cyclodextrine pour réaliser cette inclusion présente un inconvénient majeur. En effet, il s'avère que la vitamine A incluse dans la β-cyclodextrine n'est pas protégée et se dégrade même plus rapidement que la vitamine A non incluse. Ce phénomène a été prouvé par Karl-Heinz Frömming et al. (Acta Pharm. Technol. (1988) 34 (3) 152-155) au moyen de différentes techniques : spectrophotométrie UV, spectroscopie RMN, etc... Ainsi l'utilisation de la β-cyclodextrine ne semble pas pouvoir résoudre tous les problèmes techniques évoqués ci-dessus pour une utilisation cosmétique ou dermatologique de la vitamine A ou de ses esters.

Pour améliorer la stabilité de la vitamine A ou de ses esters, il a été proposé d'utiliser la γ-cyclodextrine comme molécule hôte (US 5985296). Toutefois le complexe d'inclusion de la vitamine A dans la γ-cyclodextrine présente une solubilité en milieu aqueux très médiocre, ce qui en limite sérieusement son intérêt pour une application cosmétique ou dermatologique.

Il est également connu du document EP 0649653 A1 l'utilisation de β-CD partiellement méthylées comme promoteur d'absorption dans des compositions pharmaceutiques pour l'administration transcutanée de principes actifs.

Ce document n'incite manifestement pas l'homme du métier à utiliser une β-cyclodextrine partiellement méthylée comme vecteur de pénétration de principes actifs dans la peau sans passage transcutané, en vue d'applications cosmétiques.

Par ailleurs, la demande de brevet FR 2484252 décrit un procédé de préparation de solutions aqueuses de composés organiques biologiquement actifs insolubles ou peu solubles dans l'eau, tels que la vitamine A (ou rétinol) et ses esters, par inclusion dans des β-cyclodextrines partiellement méthylées, en particulier diméthylées. Il y est précisé que ces solutions aqueuses sont stables. A titre d'exemple, un procédé est décrit pour inclure l'acétate de vitamine A dans l'heptakis-(2,6-di-O-méthyl)-β-cyclodextrine. Toutefois le complexe d'inclusion ainsi formé est ensuite incorporé dans une préparation pharmaceutique administrable par voie orale. Ce document incite l'homme du métier à administrer les vitamines liposolubles comme la vitamine A par voie orale sous forme de solutions aqueuses, le détournant ainsi d'une utilisation topique.

D'autres auteurs ont préparé des complexes d'inclusion de différents rétinoïdes et de leurs esters dans des cyclodextrines en particulier dans la 2,6-di-O-méthyt-β-cyclodextrine ou « DM-β-CD », pour réaliser des études de solubilité dans l'eau et de stabilité (S. Mu 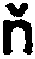 oz Botella, *et al.,* Analyst (1996) 121, 1557-1560). Des complexes d'inclusion avec le rétinol et le rétinyl acétate dans la DM-β-CD ont été préparés et étudiés. Les observations par la technique de fluorescence ont montré que ces complexes présentaient une excellente stabilité sur le long terme

Enfin, le document US 5484816 décrit la préparation d'une composition pour le traitement de la peau, dans laquelle la stabilité de la vitamine A est grandement améliorée. Cet effet positif sur la stabilité est obtenu notamment par l'inclusion de la vitamine A ou de son ester dans différentes cyctodextrines, modifiées ou non, en particulier dans une méthyl β-cyclodextrine.

Les inventeurs de la présente invention ont maintenant découvert de manière tout à fait surprenante que l'utilisation de certaines β-cyclodextrines partiellement méthylées bien déterminées, en association avec de la vitamine A ou un de ses esters classiquement utilisés en cosmétique et en dermatologie, permettait de préparer des compositions cosmétiques ou dermatologiques dont l'application conduisait à une remarquable rétention de la vitamine A ou de ses esters dans la peau, tout en évitant leur passage transdermique.

Ainsi donc, la présente invention résulte de la constatation particulièrement surprenante de ce qu'aucun passage transdermique de la vitamine A ou de son ester n'avait lieu lorsque la vitamine A ou son ester se trouvait associé dans la composition à des β-cyclodextrines partiellement méthylées bien déterminées.

Cette découverte s'avère particulièrement avantageuse puisqu'elle conduit à focaliser toute l'activité biologique de la composition sur la libération de l'agent actif au niveau de la peau qui est la zone visée selon la présente invention.

Ainsi donc, la présente invention concerne de nouvelles utilisations dans le domaine de la cosmétique et de la dermatologie d'associations de β-cyclodextrines partiellement méthylées bien déterminées avec de la vitamine A ou des esters de vitamine A.

Avant d'aborder la description proprement dite détaillée de l'invention, il semble bon de donner un certain nombre de définitions de termes employés tout au long de la présente description.

Les cyclodextrines (CD) sont des composés cycliques encore désignés par cycloamyloses ou cycloglucanes constitués d'un enchaînement de motifs glucose (6 motifs glucose pour l'α-cyclodextrine, 7 pour la β-cyclodextrine et 8 pour la γ-cyclodextrine).

Par "β-cyclodextrine diméthylée", au sens de l'invention, on entend les dérivés méthylés de la β-cyclodextrine possédant un groupe méthoxy sur, en moyenne, deux des trois positions 2, 3 et 6 de chacun des 7 motifs glucose, les groupes méthoxy étant préférentiellement situés en positions 2 et 6, la position 3 portant un groupe hydroxy. Statistiquement, les «β-cyclodextrines diméthylées » selon l'invention présentent un degré de substitution des protons des groupes hydroxy des motifs glucose par des radicaux méthyle compris entre 10 et 16, et de préférence compris entre 11 et 15.

Dans cette famille de β-cyclodextrines diméthylées, on distinguera en particulier
- le dérivé diméthylé dont tous les motifs glucose possèdent un groupe méthoxy sur chacune des positions 2 et 6, désigné sous le nom d'heptakis-(2,6-di-O-méthyl)-β-cyclodextrine ou encore DIMEB, qui présente donc un degré de substitution défini ci-dessus égal à 14, et
- parmi les produits commerciaux existant dans la famille des β-cyclodextrines diméthylées, la RAMEB qui est une β-cyclodextrine dont chaque unité glucose est statistiquement diméthylée. Le degré de substitution moyen de la RAMEB par les radicaux méthyle est d'environ 12,6, ainsi que cela est notamment indiqué dans le document WO 0145615.

Selon une première caractéristique essentielle, la présente invention concerne l'utilisation dans une composition cosmétique contenant de la vitamine A ou l'un de ses esters cosmétiquement acceptables, à titre d'agent actif, d'un dérivé méthylé de la β-cyclodextrine, dit β-cyclodextrine diméthylée, en tant qu'agent favorisant la pénétration dans l'épiderme et la rétention dans la peau dudit agent actif, ledit dérivé possédant un groupe méthoxy sur, en moyenne, deux des trois positions 2, 3 et 6 de chacun des 7 motifs glucose, les groupes méthoxy étant préférentiellement situés en positions 2 et 6, la position 3 portant un groupe hydroxy, et présentant statistiquement un degré de substitution des protons des groupes hydroxy des motifs glucose par des radicaux méthyle compris entre 10 et 16, et de préférence compris entre 11 et 15.

Selon une deuxième caractéristique, l'invention concerne un procédé de soin cosmétique caractérisé en ce qu'il comprend l'application sur la peau d'une composition cosmétique contenant l'association d'une β-cyclodextrine diméthylée et de la vitamine A ou d'au moins l'un de ses esters cosmétiquement acceptables, les composants de ladite association étant tels que définis précédemment.

Il s'agit plus précisément d'un procédé de soin cosmétique, notamment destiné à améliorer le tonus, la fermeté et l'élasticité de la peau et/ou à retarder l'apparition des rides ou en atténuer la profondeur et/ou à réaliser le soin des peaux grasses, caractérisé en ce qu'il comprend l'application sur la peau d'une composition cosmétique contenant une association de β-cyclodextrines diméthylées et de vitamine A ou d'au moins un de ses esters cosmétiquement acceptables.

Ainsi, selon sa dernière caractéristique, l'invention concerne l'utilisation d'une association de vitamine A ou d'au moins un de ses esters dermatologiquement acceptables et d'une β-cyclodextrine diméthylée, pour la fabrication d'une composition dermatologique à activité cicatrisante ou pour le traitement des peaux à tendance acnéique.

La présente invention résulte, comme exposé précédemment du fait que ses inventeurs ont mis en évidence, notamment comme cela ressort des exemples qui suivent, que le recours à une β-cyclodextrine diméthylée telle que définie précédemment permettait d'améliorer grandement l'absorption de la vitamine A ou de ses esters par la peau, tout en évitant un passage transdermique, et d'augmenter ainsi sa biodisponibilité.

Les β-cyclodextrines diméthylées utilisables selon l'invention en combinaison avec de la vitamine A ou un de ses esters peuvent être toutes les β-cyclodextrines telles que définies ci-dessus.

On peut donc utiliser, selon la présente invention, toute β-cyclodextrine diméthylée telle que définie précédemment, ces β-cyclodextrines présentant un degré de substitution compris entre 10 et 16, de préférence entre 11 et 15.

Selon une variante particulièrement avantageuse, on recourra à l'heptakis-(2,6-di-O-méthyl)-β-cyclodextrine ou DIMEB.

Selon une autre variante avantageuse de l'invention, on utilisera, à titre de β-cyclodextrines diméthylées, des β-cyclodextrines statistiquement diméthylées sur chaque glucose, couramment commercialisées sous le nom de RAMEB telles que définies ci-dessus.

L'invention pourra être mise en oeuvre avec de la vitamine A elle-même sous sa forme alcool (rétinol). Toutefois, on choisira de préférence d'utiliser ses esters. Tous les esters de la vitamine A classiquement utilisés en cosmétique et en dermatologie pourront être utilisés selon la présente invention. On choisira de préférence l'acétate et le propionate de vitamine A.

Comme cela est déjà connu dans la littérature, la vitamine A et ses esters forment aisément des complexes d'inclusion avec les β-cyclodextrines, en particulier avec les β-cyclodextrines alkylées telles que la DIMEB et la RAMEB.

Ces complexes d'inclusion pourront être utilisés pour introduire les associations de vitamine A ou d'esters de vitamine A et de β-cyclodextrines méthylées dans les compositions impliquées dans l'invention et, d'une façon générale, de la vitamine A ou son ester se retrouvera au moins partiellement inclus dans la β-cyclodextrine diméthylée.

Ainsi, pour préparer les complexes d'inclusion pour la mise en oeuvre de l'invention, on peut utiliser l'un des procédés décrits, en particulier celui décrit dans le document FR 2484252. Selon ce procédé, on dissout d'une façon générale la β-cyclodextrine diméthylée dans de l'eau puis on ajoute à la solution obtenue la vitamine A ou son ester, soit directement, soit sous forme d'une solution organique miscible à l'eau, et on évapore ensuite le solvant, de façon à récupérer le complexe d'inclusion sous forme solide.

Selon une autre variante, on peut également récupérer le complexe d'inclusion en solution avant toute évaporation, ou bien le complexe d'inclusion sous forme solide obtenu à l'étape précédente peut être solubilisé à nouveau en milieu aqueux pour son utilisation ultérieure dans le cadre de la présente invention.

Il est également possible d'utiliser les procédés de préparation décrits par S. Mu 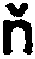 oz Botella, *et al.* (Analyst (1996) 121, 1557-1560). Par exemple, selon le principe du premier de ces procédés décrits dans cette publication, on introduit dans un ballon rotatif le rétinol dissout dans l'hexane, puis on évapore le solvant de façon à former une fine pellicule de rétinol sur les parois du ballon. On introduit alors dans le ballon une solution aqueuse de β-cyclodextrine diméthylée, puis on agite cette solution pendant 24 à 48 heures par des moyens magnétiques.

De tels procédés de préparation s'avèrent particulièrement intéressants puisqu'ils permettent d'obtenir des solutions aqueuses, le cas échéant sous forme de gels, de la vitamine A et de ses esters.

Selon un aspect de l'invention, les complexes d'inclusion définis ci-dessus peuvent être utilisés aussi bien sous forme de solutions aqueuses que sous forme de poudre sèche pour être incorporés dans des compositions cosmétiques ou dermatologiques.

Il est également apparu que la composition cosmétique ou dermatologique impliquée dans l'invention peut être préparée sous différentes formes, en particulier sous forme de gel aqueux, de lotion, d'émulsion et de poudre.

Il s'agira en particulier de produits cosmétiques de soin ou de maquillage traitant, destinés en particulier à lutter contre l'apparition des effets du vieillissement actinique ou chronologique, tels que les rides, la perte de l'élasticité et de la tonicité de la peau, ou encore destinés au soin des peaux grasses.

Il s'agira également de préparations dermatologiques, notamment destinées à favoriser la cicatrisation cutanée ou au traitement des peaux à tendance acnéique.

Les concentrations en agents actifs associés contenus dans les compositions cosmétiques ou dermatologiques de l'invention peuvent varier dans de larges gammes.

Toutefois, les compositions impliquées dans l'invention contiennent avantageusement de 0,0001 % à 0,5 % et, de préférence, de 0,001 % à 0,1 %, en poids de vitamine A ou de ses esters et de 0,0005 % à 10 % et, de préférence, de 0,005 % à 1 %, en poids de β-cyclodextrine diméthylée.

Comme exposé précédemment, les deux constituants ci-dessus sont avantageusement sous forme de complexe d'inclusion, ce complexe d'inclusion se trouvant avantageusement à une concentration comprise entre 0,005 % et 2,5 %, de préférence entre 0,005 % et 0,5 %, en poids dans ces compositions.

Selon une variante avantageuse, la proportion en poids de la vitamine A ou ses esters par rapport à la β-cyclodextrine diméthylée varie entre 0,1 et 1.

Les exemples qui suivent sont donnés à titre purement illustratif de l'invention.

### EXEMPLES

Sauf indications contraires, les concentrations sont exprimées en pourcentages en poids.

### Exemple 1 : Préparation d'un gel aqueux contenant un complexe d'inclusion du propionate de vitamine A dans la RAMEB (ou diméthyl β-cyclodextrine commerciale):

L'ester de vitamine A utilisé est le propionate de vitamine A, ci-après désigné par PVA

A 10 ml d'une solution 50 mM de RAMEB, on ajoute 200 µl d'une solution acétonique 1M de PVA, pour obtenir une concentration finale 20 mM en PVA.

On laisse sous hotte ventilée et sous agitation pendant 24 heures.

On centrifuge ensuite pendant 6 minutes à 6000 tours/minute les solutions obtenues. La solution obtenue après cette opération est limpide, de couleur jaune pâle et ne contient pas de précipité.

La phase aqueuse est filtrée, congelée et lyophilisée.

On obtient ainsi une poudre. Celle-ci peut être utilisée sous cette forme dans la préparation d'une composition cosmétique ou dermatologique, ou bien, selon les besoins, être facilement remise en solution en milieu aqueux.

Le milieu aqueux précité peut être un gel aqueux, comme par exemple un gel d'hydroxyéthylcellulose (Natrosol^{®}, Hercules Inc. Etats-Unis) à 1,8% dans l'eau.

Selon le présent exemple, on ajoute à 10 g (75%) de gel aqueux d'hydroxyéthylcellulose défini ci-dessus, 3,3 g (25%) de solution aqueuse du complexe d'inclusion préparé ci-dessus sous forme de poudre, soit une quantité de 24 mg de PVA (soit environ 0,72% de PVA final). On obtient ainsi un gel contenant le complexe d'inclusion du PVA dans de la RAMEB utilisable selon l'invention.

### Exemple 2 : Pénétration du PVA dans l'épiderme

Le but de cette étude est de comparer la pénétration et la répartition du PVA dans de l'épiderme humain frais, selon qu'il se présente sous forme d'un complexe d'inclusion avec une diméthyl β-cyclodextrine ou avec une γ-cyclodextrine, ainsi qu'incorporé dans un gel en l'absence de toute cyclodextrine.

### 1. Préparation des gels

On prépare les gels suivants.

### a) Gel de base

Ce gel est constitué d'hydroxyéthylcellulose (Natrosol^{®}, Hercules Inc. Etats-Unis) à 1,8% en poids dans l'eau.

### b) Gel contenant le complexe d'inclusion RAMEB/PVA, utilisable selon l'invention (gel RAMEB/PVA).

Le gel RAMEB est le gel préparé à l'exemple 1 ci-dessus.

### c) Gel de comparaison contenant le complexe d'inclusion de PVA et de γ-cyclodextrine (gel gamma/PVA)

Le gel gamma/PVA est constitué à partir de 10 g (75 %) de gel de base auxquels on ajoute 3 ml (25 %) d'une suspension aqueuse à 32 mg/ml de complexe d'inclusion γ-CD/PVA préparé comme indiqué ci-après, soit 24 mg de PVA théorique (soit environ 0,72 % de PVA final).

Le complexe d'inclusion γ-CD/PVA est préparé comme suit. A une solution 10 mM de γ-CD on ajoute 200 µL d'une solution acétonique de propionate de vitamine A 1M (concentration finale 20 mM). On laisse sous hotte ventilée (pour évaporer l'acétone) et sous agitation pendant 24h. Ensuite on centrifuge 6 minutes à 6000 tours/minute les solutions obtenues. Le précipité obtenu est lavé par centrifugation par 2×3 mL d'eau pour éliminer la CD libre et 2x3 mL d'éther pour éliminer le propionate de vitamine A libre, puis séché sous un courant d'azote, congelé et lyophilisé.

### d) Gel témoin/PVA

Le gel témoin/PVA est constitué de 25 g de gel de base (75 %) auxquels on ajoute d'abord un mélange de 60 mg de PVA (soit environ 0,72 % de PVA final) et de 0,83 g de Tween 60 (polyéthylène (20) sorbitan monostéarate) (soit 2,5 % de Tween 60 final) et ensuite 7,5 g d'eau.

L'étude de pénétration dans l'épiderme s'effectue à l'aide d'une cellule de Franz présentant une surface de 2 cm² et un volume de 4,2 ml. Le liquide récepteur est constitué de tampon phosphate du commerce (Sigma) (10 nM, NaCl 120 mM, KCl 2,7 mM, pH 7,4), d'azoture de sodium 0,1 % du commerce (Sigma), d'éthanol à 20 % et de solubilisant LRI (mélange commercialement disponible d'éther butylique de polyoxypropylène-polyoxyéthylène et d'huile de ricin hydrogénée polyéthylénée) à 4 %.

On prépare ensuite 15 échantillons d'épiderme à partir de 5 fragments de peau d'origine différente coupés en trois, dont on aura préalablement séparé le derme à chaud, suivant une technique classique. Ces échantillons sont montés sur 15 cellules de Franz. Au cours d'une même expérience, les 3 gels (RAMEB/PVA, gamma/PVA et témoin/PVA) sont appliqués de façon occlusive sur les échantillons, chaque gel étant appliqué sur cinq d'entre eux.

Après 24 heures de contact, l'excédent de gel à la surface cutanée est lavé à l'aide de coton-tiges. Les coton-tiges ainsi que la partie supérieure de la cellule sont immergés dans 10 ml d'isopropanol et soumis aux ultrasons pendant 15 mn puis agités durant 2 heures. Les solutions sont diluées au 1/10 et filtrées sur filtre de 0,2 µm avant dosage. Le stratum corneum est prélevé sur des pastilles adhésives par la technique dite de « stripping » au moyen d'un appareil décrit dans le document FR 2710517, Parfums Christian Dior. Sept prélèvements successifs par échantillon (pression 300 g/cm², durée 12 s) sont effectués. Les pastilles adhésives correspondant à chaque échantillon sont placées dans 2 ml de méthanol pendant 1 nuit à 4°C pour en extraire le PVA. Le milieu méthanolique est ensuite agité pendant 2 heures, puis filtré sur filtre de 0,2 µm avant dosage. L'épiderme débarrassé du stratum corneum est à son tour placé dans 1 ml de méthanol pour en extraire le PVA suivant le même mode opératoire que ci-dessus.

Les dosages du PVA sont effectués par HPLC selon une méthode classique.

Les résultats de dosage en PVA respectivement dans le stratum corneum, dans l'épiderme et dans le liquide récepteur sont regroupés dans le tableau ci-dessous dans lequel les valeurs sont exprimées en pourcentage de PVA extrait par rapport à la quantité de PVA appliqué sur l'échantillon.

| | **stratum corneum** | **épiderme** | **liquide récepteur** |
|---|---|---|---|
| gel témoin | 0,378 | 0,139 | 0,000 |
| gel RAMEB | 0,612 | 0,516 | 0,003 |
| gel gamma | 0,187 | 0,068 | 0,000 |

On constate que le pourcentage de PVA ayant pénétré dans la peau est beaucoup plus élevé pour le gel de RAMEB/PVA et, ce, quelque soit le compartiment considéré. Le gel obtenu à partir du complexe γ-CD/PVA est moins efficace que le gel témoin. Pour le gel de RAMEB/PVA, on constate également que le pourcentage de PVA dans l'épiderme est du même ordre de grandeur que celui dans le *stratum corneum,* ce qui traduit une grande efficacité en terme de pénétration. Par contre, pour les deux autres gels, le pourcentage de PVA dans l'épiderme est beaucoup plus faible.

### Exemple 3 : Influence du témoin

Une étude, identique à celle décrite dans l'exemple 2, est effectuée avec le gel RAMEB/PVA et le PVA seul en milieu huileux.

Le milieu huileux est une huile de carnation du commerce contenant 0,75 % de PVA. Les résultats de dosage en PVA dans les différents compartiments de la peau montrent que l'on dose dix fois plus de PVA dans le liquide récepteur lorsque celui-ci est sous forme de complexe d'inclusion RAMEB/PVA. On peut donc en conclure que le gel de RAMEB/PVA permet une excellente pénétration du PVA dans l'épiderme comparativement au PVA seul, quelque soit la formulation de ce dernier.

### Exemple 4 : Pénétration du PVA dans la peau humaine

Cette étude, réalisée sur peau humaine fraîche, est destinée à comparer la pénétration et la répartition du PVA au travers du revêtement cutané humain *(stratum corneum,* épiderme et derme) et en fonction du gel.

Les gels utilisés sont le gel témoin/PVA et le gel de RAMEB/PVA tels décrits dans l'exemple 2.

L'étude s'effectue à l'aide de cellules de Franz caractérisées plus haut.

Le liquide récepteur est de même composition que celui utilisé à l'exemple 2.

Au cours d'une même expérience, les 2 gels sont appliqués suivant la même méthode qu'à l'exemple 2 sur 24 échantillons provenant de 8 fragments de peau d'origine différente.

Après 24 heures de contact, l'excédent de gel à la surface cutanée est lavé à l'aide de coton-tiges.

Les coton-tiges ainsi que la partie supérieure de la cellule sont immergés dans 10 ml d'isopropanol et soumis aux ultrasons pendant 15 minutes puis agités durant 2 heures.

Les solutions sont diluées au 1/10 et filtrées sur filtre de 0,2 µm avant dosage.

Le stratum corneum est recueilli par 7 strippings successifs (pression 300 g/cm², durée 12 s). Le PVA est extrait par solubilisation dans 2 ml de méthanol pendant 1 nuit à 4°C puis agitation 2 heures et enfin filtration sur filtre de 0,2 µm avant dosage.

Une fois le stratum corneum retiré, on sépare l'épiderme du derme à l'aide d'un scalpel.

Le PVA est extrait de l'épiderme par solubilisation de ce dernier dans 1 ml de méthanol pendant 1 nuit à 4°C puis agitation 2 heures et enfin filtration sur filtre de 0,2 µm avant dosage.

Les dosages du PVA sont effectués par HPLC.

Les dosages en PVA dans les différents compartiments de la peau ont été effectués. Aucune trace de PVA n'a été détectée dans le liquide récepteur contrairement aux résultats obtenus dans l'exemple 2.

Le gel RAMEB/PVA ne favorise donc pas le passage transdermique du PVA.

### Exemple 5 : Compositions dermatologiques et cosmétiques

Dans les exemples de formulation de compositions selon l'invention, on utilise, à titre de principe actif, le complexe RAMEB/PVA préparé selon l'exemple 1.
a) Gel dermatologique cicatrisant

| | |
|---|---|
| Glycol | 3 |
| Sepigel 305 | 3 |
| Solubilisant Cremophor CO 60 | 2 |
| PEG (Pluriol E 1505) | 1,5 |
| Conservateur | 0,5 |
| Parfum | 0,3 |
| Eau | 89,2 |
| Complexe RAMEB/PVA | 0,5 |

b) Crème de jour anti-rides (émulsion)

| | |
|---|---|
| Brij 721 | 2,5 |
| Tegin 90 | 1,1 |
| Alcool stéarylique | 5 |
| Tricaprat/caprylate glycérol | 20 |
| Butylèneglycol | 3 |
| Glycérol | 2 |
| Conservateur | 0,5 |
| Parfum | 0,5 |
| Eau | 64,9 |
| Complexe RAMEB/PVA | 0,5 |

c) Lotion revitalisante

| | |
|---|---|
| Butylèneglycol | 3 |
| EDTA | 0,1 |
| Solubilisant | 1 |
| Parfum | 0,3 |
| Alcool | 5 |
| Eau | 90,4 |
| Complexe RAMEB/PVA | 0,2 |

d) Poudre pour le soin des peaux grasses

| | |
|---|---|
| Talc | 20 |
| Mica | 20 |
| Séricite | 20 |
| Pigments | 8 |
| Poudre organique (Nylon) | 20 |
| Silice | 8,75 |
| Huile minérale ou silicone | 3 |
| Complexe RAMEB/PVA | 0,25 |

## Revendications

1. Utilisation, dans une composition cosmétique contenant de la vitamine A ou l'un de ses esters cosmétiquement acceptables, à titre d'agent actif, d'un dérivé méthylé de la β-cyclodextrine, dit β-cyclodextrine diméthylée, en tant qu'agent favorisant la pénétration dans l'épiderme et la rétention dans la peau dudit agent actif, ledit dérivé possédant un groupe méthoxy sur, en moyenne, deux des trois positions 2, 3 et 6 de chacun des 7 motifs glucose, les groupes méthoxy étant préférentiellement situés en positions 2 et 6, la position 3 portant un groupe hydroxy, et présentant statistiquement un degré de substitution des protons des groupes hydroxy des motifs glucose par des radicaux méthyle compris entre 10 et 16, et de préférence compris entre 11 et 15.

2. Utilisation selon la revendication 1, **caractérisée en ce que** ladite cyclodextrine diméthylée est essentiellement constituée d'heptakis-2,6-di-O-méthyl-β-cyclodextrine, encore connue sous le nom de DIMEB.

3. Utilisation selon la revendication 1, **caractérisée en ce que** ladite cyclodextrine diméthylée est essentiellement une β-cyclodextrine statistiquement diméthylée sur chaque motif glucose, communément dénommée RAMEB, ayant un degré de substitution de l'ordre de 12,6.

4. Utilisation selon l'une des revendications 1 à 3, **caractérisée en ce que** ledit ester de vitamine A est choisi dans le groupe constitué de l'acétate et du propionate de vitamine A.

5. Utilisation selon l'une des revendications 1 à 4, **caractérisée en ce que** la vitamine A ou l'un de ses esters et la cyclodextrine diméthylée sont introduits dans la composition cosmétique sous la forme d'un complexe d'inclusion de ladite vitamine A ou de son ester avec ladite β-cyclodextrine diméthylée.

6. Utilisation selon l'une des revendications 1 à 5, **caractérisée en ce que** ledit agent actif est au moins partiellement inclus dans une β-cyclodextrine diméthylée.

7. Utilisation selon l'une des revendications 1 à 6, **caractérisée en ce que** la composition est sous la forme d'un gel, d'une lotion, d'une émulsion ou d'une poudre.

8. Utilisation selon l'une des revendications 1 à 7, **caractérisée en ce que** la composition est une composition cosmétique contenant de 0,0001 % à 0,5 % et, de préférence, de 0,001 % à 0,1 %, en poids de vitamine A ou d'un de ses esters et de 0,0005 % à 10 % et, de préférence de 0,005 % à 1 % en poids de β-cyclodextrine diméthylée, les différents pourcentages étant exprimés en poids par rapport au poids total de ladite composition.

9. Utilisation selon la revendication 8, **caractérisée en ce que** ladite composition contient de 0,0005 % à 2,5 %, de préférence de 0,005 % à 0,5 %, en poids de complexe d'inclusion de la vitamine A ou de son ester dans la β-cyclodextrine diméthylée.

10. Procédé de soin cosmétique, **caractérisé en ce qu'**il comprend l'application sur la peau d'une composition cosmétique contenant l'association d'une β-cyclodextrine diméthylée et de la vitamine A ou d'au moins l'un de ses esters cosmétiquement acceptables, les composants de ladite association étant tels que définis dans l'une des revendications 1 à 6.

11. Procédé de soin cosmétique selon la revendication 10, **caractérisé en ce qu'**il est destiné à améliorer le tonus, la fermeté et l'élasticité de la peau et/ou à retarder l'apparition des rides ou en atténuer la profondeur et/ou à réaliser le soin des peaux grasses.

12. Utilisation d'une association de vitamine A ou d'au moins l'un de ses esters dermatologiquement acceptables et d'une β-cyclodextrine diméthylée, les composants de ladite association étant tels que définis dans l'une des revendications 1 à 6, pour la fabrication d'une composition dermatologique à activité cicatrisante ou pour le traitement des peaux à tendance acnéique.

## Claims

1. Use, in a cosmetic composition containing vitamin A or one of its cosmetically acceptable esters as the active ingredient, of a methylated β-cyclodextrin derivative, called dimethylated β-cyclodextrin, as an agent for promoting the penetration of said active ingredient into the epidermis, and its retention in the skin, said derivative possessing a methoxy group in, on average, two of the three positions 2, 3 and 6 of each of the 7 glucose units, the methoxy groups preferably being located in positions 2 and 6, and position 3 carrying a hydroxyl group, and statistically having a degree of substitution of the hydroxyl group protons of the glucose units by methyl radicals of between 10 and 16 and preferably of between 11 and 15.

2. Use according to claim 1, **characterized in that** said dimethylated cyclodextrin essentially consists of heptakis-2,6-di-O-methyl-β-cyclodextrin, also known as DIMEB.

3. Use according to claim 1, **characterized in that** said dimethylated cyclodextrin is essentially a β-cyclodextrin that is randomly dimethylated on each glucose unit with a degree of substitution in the order of 12.6, said dimethylated β-cyclodextrin commonly being known as RAMEB.

4. Use according to one of claims 1 to 3, **characterized in that** said vitamin A ester is selected from the group consisting of vitamin A acetate and propionate.

5. Use according to one of claims 1 to 4, **characterized in that** the vitamin A or one of its esters and the dimethylated cyclodextrin are introduced into the cosmetic composition in the form of an inclusion complex of said vitamin A or its ester with said dimethylated β-cyclodextrin.

6. Use according to one of claims 1 to 5, **characterized in that** said active ingredient is at least partially included in a dimethylated β-cyclodextrin.

7. Use according to one of claims 1 to 6, **characterized in that** the composition is in the form of a gel, a lotion, an emulsion or a powder.

8. Use according to one of claims 1 to 7, **characterized in that** the composition is a cosmetic composition containing from 0.0001% to 0.5% by weight, preferably from 0.001% to 0.1% by weight, of vitamin A or one of its esters and from 0.0005% to 10% by weight, preferably from 0.005% to 1% by weight, of dimethylated β-cyclodextrin, the different percentages being expressed by weight based on the total weight of said composition.

9. Use according to claim 8, **characterized in that** said composition contains from 0.0005% to 2.5% by weight, preferably from 0.005% to 0.5% by weight, of an inclusion complex of vitamin A or its ester in dimethylated β-cyclodextrin.

10. Method of cosmetic care, **characterized in that** it comprises the application to the skin of a cosmetic composition containing the combination of a dimethylated β-cyclodextrin and vitamin A or at least one of its cosmetically acceptable esters, the components of said combination being as defined in one of claims 1 to 6.

11. Method of cosmetic care according to claim 10, **characterized in that** it is intended to improve the tone, firmness and elasticity of the skin, and/or to delay the appearance of wrinkles or reduce their depth, and/or to care for greasy skin.

12. Use of a combination of vitamin A or at least one of its dermatologically acceptable esters and a dimethylated β-cyclodextrin, the components of said combination being as defined in one of claims 1 to 6, for the manufacture of a dermatological composition with healing activity or for treating skin prone to acne.

## Patentansprüche

1. Verwendung in einer kosmetischen Zusammensetzung, die Vitamin A oder einen seiner kosmetisch verträglichen Ester als Wirkstoff enthält, eines methylierten Derivat von β-Cyclodextrin, genannt dimethyliertes β-Cyclodextrin, als Mittel, das die Penetrierung in die Epidermis und den Rückhalt in der Haut des Wirkstoffs begünstigt, wobei das Derivat eine Methoxygruppe an im Mittel zwei der drei Positionen 2, 3 und 6 von jedem der 7 Glucosemotive aufweist, wobei die Methoxygruppen vorzugsweise in den Positionen 2 und 6 liegen, wobei die Position 3 eine Hydroxygruppe trägt, und wobei es statistisch einen Substitutionsgrad der Protonen der Hydroxygruppen der Glucosemotive durch Methylreste zwischen 10 und 16 und vorzugsweise zwischen 11 und 15 aufweist.

2. Verwendung nach Anspruch 1, **dadurch gekennzeichnet, daß** das dimethylierte Cyclodextrin im wesentlichen aus Heptakis-2,6-Di-O-Methyl-β-Cyclodextrin, auch bekannt unter dem Namen DIMEB, besteht.

3. Verwendung nach Anspruch 1, **dadurch gekennzeichnet, daß** das dimethylierte Cyclodextrin im wesentlichen ein β-Cyclodextrin ist, das statistisch auf jedem Glucosemotiv dimethyliert ist, allgemein bezeichnet als RAMEB mit einem Substitutionsgrad in der Größenordnung von 12,6.

4. Verwendung nach einem der Ansprüche 1 bis 3, **dadurch gekennzeichnet, daß** der Ester von Vitamin A gewählt ist aus der Gruppe, die besteht aus Acetat und Propionat von Vitamin A.

5. Verwendung nach einem der Ansprüche 1 bis 4, **dadurch gekennzeichnet, daß** das Vitamin A oder einer seiner Ester und dimethyliertes Cyclodextrin eingeführt werden in die kosmetische Zusammensetzung in Form eines Einschlußkomplexes des Vitamins A oder seines Esters mit dem dimethylierten β-Cyclodextrin.

6. Verwendung nach einem der Ansprüche 1 bis 5, **dadurch gekennzeichnet, daß** der Wirkstoff wenigstens teilweise in einem dimethylierten β-Cyclodextrin umfaßt ist.

7. Verwendung nach einem der Ansprüche 1 bis 6, **dadurch gekennzeichnet, daß** die Zusammensetzung in Form eines Gels, einer Lotion, einer Emulsion oder eines Pulvers vorliegt.

8. Verwendung nach einem der Ansprüche 1 bis 7, **dadurch gekennzeichnet, daß** die Zusammensetzung eine kosmetische Zusammensetzung ist, die 0,0001 bis 0,5 Gewichtsprozent und vorzugsweise 0,001 bis 0,1 Gewichtsprozent Vitamin A oder einen seiner Ester und 0,0005 bis 10 Gewichtsprozent, vorzugsweise 0,005 bis 1 Gewichtsprozent dimethyliertes β-Cyclodextrin enthält, wobei die verschiedenen Prozentanteile in Gewicht bezüglich dem Gesamtgewicht dieser Zusammensetzung ausgedrückt sind.

9. Verwendung nach Anspruch 8, **dadurch gekennzeichnet, daß** die Zusammensetzung 0,0005 bis 2,5 Gewichtsprozent, vorzugsweise 0,005 bis 0,5 Gewichtsprozent eines Einschlußkomplexes des Vitamins A oder seines Esters in dem dimethylierten β-Cyclodextrin enthält.

10. Verfahren zur kosmetischen Pflege, **dadurch gekennzeichnet, daß** es die Anwendung auf die Haut von einer kosmetischen Zusammensetzung umfaßt, die die Zuordnung eines dimethylierten β-Cyclodextrins und des Vitamins A oder wenigstens eines seiner kosmetisch verträglichen Ester umfaßt, wobei die Bestandteile der Zuordnung wie in einem der Ansprüche 1 bis 6 definiert sind.

11. Verfahren zur kosmetischen Pflege nach Anspruch 10, **dadurch gekennzeichnet, daß** es dazu vorgesehen ist, den Tonus, die Festigkeit und Elastizität der Haut zu verbessern und/oder das Auftreten von Falten zu verzögern oder deren Tiefe zu mindern und/oder die Pflege fetter Haut auszuführen.

12. Verwendung einer Zuordnung von Vitamin A oder wenigstens eines seiner dermatologisch verträglichen Ester und eines dimethylierten β-Cyclodextrins, wobei die Bestandteile dieser Zuordnung sowie in einem der Ansprüche 1 bis 6 definiert sind, zur Herstellung einer dermatologischen Zusammensetzung mit vernarbender Wirkung oder zur Behandlung der Haut, die eine Tendenz zu Aknen aufweist.
